# EUROPÄISCHE PATENTSCHRIFT

(11) **EP 1 864 628 B1**
(45) Veröffentlichungstag und Bekanntmachung des Hinweises auf die Patenterteilung: **14.10.2009**
(21) Anmeldenummer: 07450103.2
(22) Anmeldetag: 06.06.2007
(51) Int. Cl.: A61D 19/04, A61B 17/435

(54) **Dosiereinrichtung für den uterinen Embryotransfer**
Metering device for uterine embryo transfer
Dispositif de dosage pour le transfert d'embryons dans l'utérus

(30) Priorität: 08.06.2006 AT 9812006
(43) Veröffentlichungstag der Anmeldung: 12.12.2007
(73) Patentinhaber: Steiner, Hans-Peter, 8010 Graz (AT)
(72) Erfinder: Steiner, Hans-Peter, 8010 Graz (AT)
(74) Vertreter: Babeluk, Michael

(56) Entgegenhaltungen:
- FR-A1- 2 683 140
- US-A- 4 257 268
- US-A1- 2004 210 200

## Beschreibung

Die Erfindung betrifft eine Dosiereinrichtung für den uterinen Embryotransfer mit einer Halteeinrichtung für eine Kolbenspritze, welche mit dem proximalen Ende eines Embryotransfer-Katheters verbindbar ist, wobei der Spritzenkolben der Kolbenspritze an einem verschiebbaren Betätigungselement lösbar befestigt ist, wobei die Halteeinrichtung der Kolbenspritze an einem mit einer Hand fassbaren Griffelement befestigt ist, in welchem ein fingerbetätigbares Abzugelement drehbar gelagert ist, und wobei das Abzugelement über ein Getriebe mit dem verschiebbaren Betätigungselement des Spritzenkolbens verbunden ist.

Ein wesentlicher Schritt, welcher über Erfolg oder Misserfolg einer In-Vitro-Fertilisierung (IVF) entscheidet, ist der Embryonentransfer. Die Embryonen müssen dabei aus einem Gefäß mit einer Nährlösung, beispielsweise einer Petrischale, in einen Embryotransfer-Katheter aufgenommen und nach der genauen Positionierung des Katheters möglichst schonend in den Uterus entlassen werden. Die Aufnahme der Embryonen aus der Nährlösung erfordert großes handwerkliches Geschick und muss unter dem Mikroskop durchgeführt werden. Dabei wird zunächst in den mit einer Transferlösung gefüllten Katheter eine geringe Menge Luft eingesaugt, danach eine Portion Nährlösung mit zumindest einem Embryo und schließlich wieder eine kleine Luftmenge, so dass nach der Aufnahme der Embryonen im vorderen Bereich des Embryotransfer-Katheters eine Abfolge aus Luft, Nährlösung samt Embryonen, Luft und Transferlösung anzutreffen ist. Naturgemäß sind die eingesetzten Mengen an Transferlösung, Nährlösung und Luft je nach Anwender unterschiedlich.

Weltweit werden schätzungsweise über 90 % der uterinen Embryotransfers lediglich mittels einer Tuberkulinspritze und diversen Kathetern durchgeführt. Für den Reproduktionsmediziner ergibt sich bei der Betätigung des Spritzenkolbens das Problem, dass es im Moment der Überwindung der Kolbenhaftreibung zu einer unkontrollierten, plötzlichen und somit nicht exakt dosierbaren Bewegung kommt. Dadurch besteht die Gefahr, dass ein zu großes Volumen der Transferlösung mit zu rascher Geschwindigkeit in die Gebärmutter gespült wird und es dadurch zu zwei gefürchteten Komplikationen kommt:
- Die Nährlösung mit den Embryonen wird in Richtung Gebärmutterhals gespült - die Embryonen sind dadurch verloren.
- Die Nährlösung mit den Embryonen wird in Richtung Eileiterostium und in den Eileiter gespült - es besteht die Gefahr einer Eileiterschwangerschaft.

Zu einem geringen Prozentsatz werden die kleinsthubigen Saug- und Druckbewegungen (sowohl bei der Aufnahme des Embryos als auch beim Einbringen des Embryos in den Uterus) mit am Markt befindlichen Dosiereinrichtungen bewerkstelligt, welche im Wesentlichen aus einer Halterung für eine Kolbenspritze bestehen, deren Kolben mit einer Gewindestange zusammenwirkt, wobei über eine Rändelschraube kleine Hubbewegungen des Kolbens bewerkstelligt werden können. Beispielsweise wird dafür ein Microinjector (z.B. Narishige IM-6, Tokio, Japan) verwendet, bei welchem mit einer vollen Rotation der Rändelschraube ein Volumen von 10 µl eingesaugt werden kann. Das Gerät arbeitet zwar präzise, ist aber sehr unhandlich und schwer und muss - insbesondere bei der Abgabe der Embryonen in den Uterus - von einer Hilfsperson des IVF-Mediziners betätigt werden.

Ähnliches gilt für ein weiteres bekanntes Dosiergerät (Fa. AstroMedTec, Salzburg, Österreich), welches eine Halteeinrichtung für eine Kolbenspritze aufweist, wobei der Spritzenkolben durch eine Schraube fixierbar ist. Über eine seitlich angeordnete Rändelschraube, die mit einer Gewindestange zusammenwirkt, kann bei einer vollen Umdrehung ein Volumen von 9 µl eingesaugt bzw. abgegeben werden.

Aus der FR 2 683 140 A1 ist eine Dosiereinrichtung gemäß dem Oberbegriff des Anspruchs 1, mit einer Halteeinrichtung für eine Kolbenspritze bekannt, welche mit dem proximalen Ende eines Embryotransfer-Katheters verbindbar ist, wobei der Spritzenkolben der Kolbenspritze an einem verschiebbaren Betätigungselement befestigt ist. Die Halteeinrichtung der Kolbenspritze ist an einem mit einer Hand fassbaren Griffelement befestigt, in welchem ein Abzugelement drehbar gelagert ist, wobei das Abzugelement über ein Getriebe mit dem verschiebbaren Betätigungselement des Spritzenkolbens verbindbar ist. Der Betätigungshebel der FR 2 683 140 kann allerdings nicht durch eine einfache Fingerbewegung dosiert von einer Druck- zu einer Saugbewegung umgeschaltet werden, ohne die Griffposition an der Dosiereinrichtung zu ändern.

Die GB 1 430 045 A beschreibt eine Vorrichtung zur Empfängnisverhütung mit einer Halteeinrichtung für eine Kolbenspritze, wobei der Spritzenkolben der Kolbenspritze an einem verschiebbaren Betätigungselement lösbar befestigt ist. Die Halteeinrichtung der Kolbenspritze ist an einem mit einer Hand fassbaren Griffelement befestigt, in welchem ein fingerbetätigbares Abzugelement drehbar gelagert ist.

Im Zusammenhang mit der dosierten Abgabe von Medikamenten aus Kolbenspritzen sind auch eine Reihe von Vorrichtungen bekannt geworden, bei welchen der Kolben elektromotorisch und programmgesteuert betrieben wird. So ist beispielsweise aus der EP 0 270 628 B eine elektromechanische Vorrichtung zum einwegigen Ausdrücken von Injektionsspritzen bekannt, welche die Form einer Spritzpistole aufweist, deren Abzugelement einen im Gehäuse integrierten Elektromotor samt Getriebe steuert. Über eine Gewindestange wird ein Mitnehmer betrieben, der auf das Ende des Spritzenkolbens einwirkt. Die genannte Vorrichtung ist aus mehreren Gründen für die In-Vitro-Fertilisierung nicht geeignet. So sind derartige Motorspritzen nur für die Abgabe eines Medikamentes, d.h. eine Bewegung des Kolbenstempels in Druckrichtung, nicht jedoch in Saugrichtung geeignet. Weiters könnte selbst bei Vorhandensein einer Umschalteinrichtung der jeweils vom Kolben zurückgelegte Weg (und damit das vom Kolben bewegte Volumen) nur schwer abgeschätzt werden. Bei der IVF ist es im Gegensatz notwendig, sowohl bei der Aufnahme der Embryonen aus der Petrischale als auch bei deren Abgabe in den Uterus flexibel und angepasst an die jeweilige Situation reagieren zu können, wobei jederzeit eine Rückmeldung über die im Embryotransfer-Katheter bewegten Volumina notwendig ist.

Aufgabe der vorliegenden Erfindung ist es daher, ausgehend von bekannten Dosiervorrichtungen für den uterinen Embryotransfer, Verbesserungen vorzuschlagen, die es ermöglichen die Dosiervorrichtung möglichst ohne Hilfspersonal nur mit einer Hand betätigen zu können und gleichzeitig die Aufnahme der Embryonen aus der Nährlösung sowie die Abgabe in den Uterus optimal dosieren und überwachen zu können.

Diese Aufgabe wird erfindungsgemäß dadurch gelöst, dass das Abzugelement zur Unterstützung von Drehbewegungen in beide Drehrichtungen, welche Druck- bzw. Saugbewegungen des Spritzenkolbens auslösen, eine Öffnung für einen das Abzugelement betätigenden Finger (vorzugsweise Zeigefinger) aufweist. Durch diese erfindungsgemäße Maßnahme kann der IVF-Mediziner ohne Änderung der Griffposition an der Dosiereinrichtung von einer Druckbewegung auf eine Saugbewegung umschalten. Dies ist oft dann notwendig, wenn bei der Aufnahme der Embryonen aus der Nährlösung ein Fehlversuch stattfindet und die Prozedur neu gestartet werden muss. Im Unterschied zu vielen bekannten Dosiervorrichtungen, bei welchen von einer Hilfsperson eine Rändelschraube nach den Anweisungen des IVF-Mediziners gedreht werden muss, ist bei der erfindungsgemäßen Dosiereinrichtung keine Assistenz notwendig. Der IVF-Mediziner kann mit einer Hand die Dosiereinrichtung betätigen, wobei deren drehbar gelagertes Abzugelement in kleinen Schritten vor- und zurückbewegt werden kann und durch das vorhandene Getriebe kleinste Volumina genau dosiert werden können. Weiters ist eine direkte Rückmeldung über die bewegten Volumina aufgrund der Stellung des Abzugelementes gegeben.

Die eingangs angeführten Probleme (Verlust der Embryonen bzw. Gefahr einer Eileiterschwangerschaft) werden mit der gegenständlichen Dosiereinrichtung weitestgehend umgangen, da der Arzt mit deren Hilfe eine exakt definierbare Menge mit einer kontinuierlichen Geschwindigkeit in die Gebärmutter spülen kann. Weiters wird die Entstehung kleinster Luftbläschen in der Nährlösung oder der Transferlösung verhindert, die bei den bisher üblichen bzw. unvermeidbaren, raschen Injektionsbewegungen oder Aufsaugbewegungen aufgetreten sind.

Gemäß einer vorteilhaften Ausgestaltung der Erfindung ist vorgesehen, dass das Abzugelement ein federbelastetes Rastelement aufweist, welches bei Betätigung des Abzugelementes mit feststehenden Tastmarken oder Rastkerben des Griffelementes hörbar und fühlbar zusammenwirkt. Der Anwender hat dadurch auch ohne Blickkontakt zur Dosiereinrichtung, beispielsweise beim Blick durch das Mikroskop bei der Embryonenaufnahme oder bei der Kontrolle der Positionierung des Embryotransfer-Katheters am Bildwandler, eine Rückmeldung über die im Katheter bewegten Volumina.

Weiters weist das Griffelement der Dosiereinrichtung erfindungsgemäß eine Führung zur Aufnahme einer längs der Achse der Kolbenspritze verschiebbaren Halterung für den auf die Kolbenspritze aufsteckbaren Embryotransfer-Katheter auf, wobei für deren Festlegung im Griffelement eine fingerbetätigbare Fixiereinrichtung vorgesehen ist.

Zur Beleuchtung des Operationsfeldes kann im Griffelement eine in Richtung des Embryotransfer-Katheters wirkende, batteriebetriebene Beleuchtungseinrichtung integriert sein. Dadurch ist das Operationsfeld - Scheide und Muttermund - der Gebärmutter optimal ausgeleuchtet.

Bisher war es üblich, eine Operationslampe zu verwenden, deren Lichtkegel nicht selten durch den Kopf oder die Schultern des IVF-Mediziners verdeckt wurde, was die Sicht auf den Muttermund erschwert hat.

Durch die lichtstarke Beleuchtungseinrichtung (z.B. Kryptonlampe mit Reflektor) ist eine ausreichende Beleuchtung des Embryotransfer-Bereiches gewährleistet, die dem sensiblen und bewegenden Moment dieser Prozedur des Embryotransfers Rechnung trägt.

Die Erfindung wird im Folgenden anhand von Beispielen näher erläutert. Es zeigen:
- Fig. 1: die erfindungsgemäße Dosiereinrichtung für den uterinen Embryo-transfer in einer Schnittdarstellung gemäß Linie A-A in Fig. 2,
- Fig. 2: die erfindungsgemäße Dosiereinrichtung gemäß Fig. 1 in einer Schnittdarstellung gemäß Linie B-B in Fig. 1, sowie
- Fig. 3: eine dreidimensionale Darstellung der Dosiereinrichtung gemäß Fig. 1 und Fig. 2 mit abgenommener Griffhälfte.

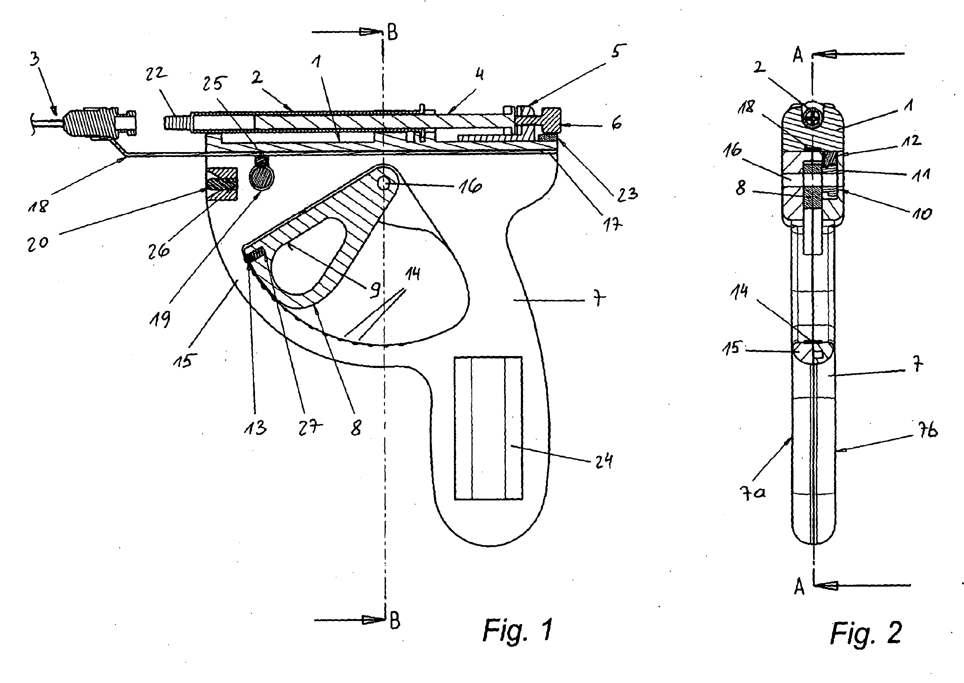

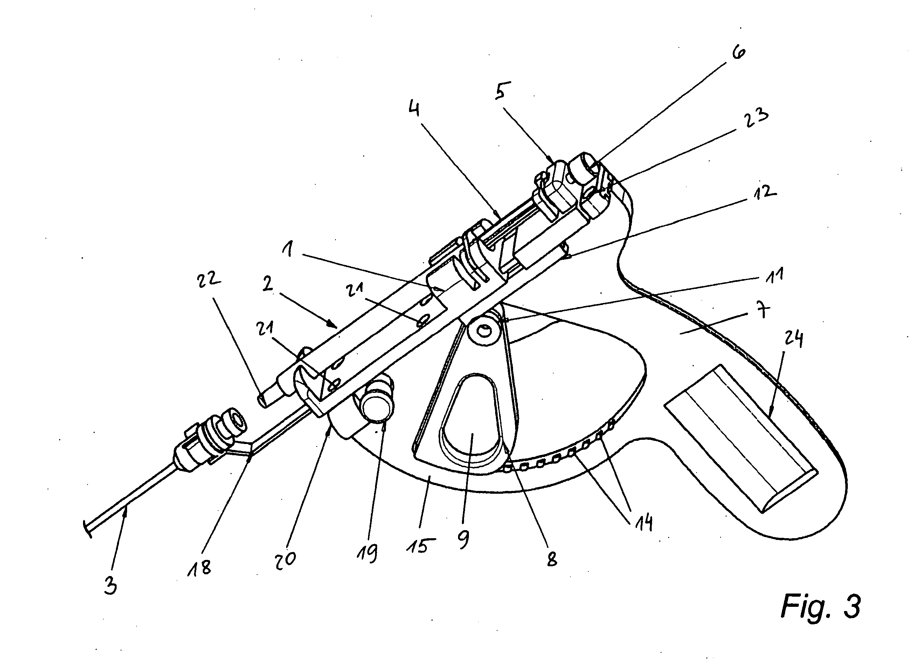

Die in den Fig. 1 bis Fig. 3 dargestellte Dosiereinrichtung für den uterinen Embryotransfer weist eine Halteeinrichtung 1 für eine Kolbenspritze 2 auf, welche mit dem proximalen Ende eines Embryotransfer-Katheters 3 verbindbar ist. Der Spritzkolben 4 der Kolbenspritze 2 ist an einem verschiebbaren Betätigungselement 5 lösbar (siehe Fixierschraube 6) befestigt. Die Halteeinrichtung 1 der Kolbenspritze 2 ist an einem Griffelement 7 befestigt, welches die Form einer Spritzpistole aufweist und mit einer Hand fassbar ist. Im Griffelement 7 ist ein Abzugelement 8 drehbar gelagert, wobei im Abzugelement 8 eine Öffnung 9 für den Zeigefinger vorgesehen ist, wodurch das Abzugelement 8 in beide Richtungen bewegt werden kann, ohne die Griffposition der Hand bzw. des Fingers zu verändern. Das Abzugelement 8 ist über ein Getriebe 10 formschlüssig mit dem Betätigungselement 5 des Spritzenkolbens 4 verbunden. Im dargestellten Beispiel besteht das Getriebe 10 aus einem am Abzugelement 8 fixierten Zahnrad 11, welches in eine Zahnstange 12 eingreift, die am verschiebbaren Betätigungselement 5 für den Spritzenkolben 4 befestigt oder einstückig mit dem Betätigungselement 5 ausgeführt ist. Durch eine Drehbewegung des Abzugelementes 8 entgegen dem Uhrzeigersinn kann somit eine Druckbewegung des Spritzenkolbens 4 ausgelöst werden, durch eine Bewegung im Uhrzeigersinn eine Saugbewegung. Mit dem Getriebe 10 lässt sich gemäß Verhältnis der Länge des Abzugelementes 8 zum Radius des Zahnrades 11 ein Untersetzungsverhältnis größer 5:1, vorzugsweise im Bereich von 10:1, realisieren. Eine Bewegung des Abzugelementes 8 zwischen zwei Rastkerben 14 bewegt ein Volumen von ca. 10 µl das sind im Embryotransfer-Katheter 3 - je nach Innendurchmesser des Katheters - ca. 1,5 bis 3,5 cm. Die Nullstellung sowie jede vierte Rastkerbe 14 kann tiefer ausgeführt sein, sodass ein lauteres "Klick"-Geräusch nach 40 µl, 80 µl und 120 µl ertönt.

Das Abzugelement 8 weist erfindungsgemäß ein durch eine Feder 27 belastetes Rastelement 13 auf, welches bei jeder Betätigung des Abzugelementes 8 mit Tastmarken oder Rastkerben 14 des Griffelementes 7 hörbar und fühlbar zusammenwirkt. Wie im dargestellten Ausführungsbeispiel dargestellt, sind die Tastmarken bzw. Rastkerben 14 auf der Innenseite eines im Griffelement 7 integrierten Bügels 15 angeordnet, welcher die Bewegungsbahn des Abzugelementes 8 nachzeichnet. Es wäre allerdings auch möglich, derartige Rastelemente im Bereich des Lagers 16 des Abzugelementes 8 vorzusehen.

Durch die Erfindung wird das Konzept *"Feel*/*See*/*Hear"* umgesetzt. Das Einrasten bzw. Überstreichen der Rastkerben 14 durch das Rastelement 13 kann vom IVF-Mediziner gehört und auch gefühlt werden, weiters gibt auch die sichtbare Stellung des Abzugelementes Auskunft darüber, welche Volumina im Embryotransfer-Katheter 3 in welche Richtung transportiert wurden.

Das Getriebe 10 besteht aus einem am Abzugelement 8 fixierten Zahnrad 11, welches in eine am verschiebbaren Betätigungselement 5 für den Spritzenkolben 4 befestigte Zahnstange 12 eingreift. Die Bewegung des Betätigungselementes 5 wird durch einen Anschlag 23 am hinteren Griffende begrenzt.

Das Griffelement 7 weist weiters eine Führung 17 zur Aufnahme einer längs der Achse der Kolbenspritze 2 verschiebbaren Halterung 18 für den auf die Kolbenspritze 2 aufsteckbaren Embryotransfer-Katheter 3 auf. Dabei wird der Katheter 3 mit seinem proximalen Ende auf den Luer-Anschluss 22 der Kolbenspritze 2 aufgesteckt und die Halterung 18 danach in dieser Stellung mit Hilfe der im Griffelement 7 angeordneten, fingerbetätigbaren Fixiereinrichtung 19 festgelegt. Die Fixiereinrichtung 19 weist eine Kugel 25 auf, die durch Betätigung eines beidseitig am Griffelement 7 vorstehenden Knopfes in Richtung Halterung 18 gedrückt oder gelöst werden kann.

Die erfindungsgemäße Dosiereinrichtung kann mit einer Vielzahl am Markt erhältlicher ein- oder zweiteiliger Embryotransfer-Katheter 3 betrieben werden, da praktisch alle derartigen Katheter einen Luer-Anschluss aufweisen der auf den Luer-Anschluss 22 der Kolbenspritze 2 aufgesteckt werden kann.

Die dargestellte Ausführungsvariante der Dosiereinrichtung weist weiters eine in Richtung des Embryotransfer-Katheters 3 wirkende, batteriebetriebene Beleuchtungseinrichtung 20 mit einem Reflektor 26 auf, welche in das Griffelement 7 integriert ist. Mit 24 ist ein Batteriefach gekennzeichnet, welches im Griffelement 7 angeordnet ist.

Besonders einfach kann die Dosiereinrichtung spritzgusstechnisch hergestellt werden, wobei beispielsweise das Griffelement 7 aus zwei miteinander verschraubbaren Kunststoff-Spritzgussteilen 7a, 7b bestehen kann, an welchen die Halteeinrichtung 1 für die Kolbenspritze 2 mittels Verschraubung 21 oder Verklebung befestigt ist.

## Patentansprüche

1. Dosiereinrichtung für den uterinen Embryotransfer mit einer Halteeinrichtung (1) für eine Kolbenspritze (2), welche mit dem proximalen Ende eines Embryotransfer-Katheters (3) verbindbar ist, wobei der Spritzenkolben (4) der Kolbenspritze (2) an einem verschiebbaren Betätigungselement (5) lösbar befestigt ist, wobei die Halteeinrichtung (1) der Kolbenspritze (2) an einem mit einer Hand fassbaren Griffelement (7) befestigt ist, in welchem ein fingerbetätigbares Abzugelement (8) drehbar gelagert ist, und wobei das Abzugelement (8) über ein Getriebe (10) mit dem verschiebbaren Betätigungselement (5) des Spritzenkolbens (4) verbunden ist, **dadurch gekennzeichnet, dass** das Abzugelement (8) zur Unterstützung von Drehbewegungen in beide Drehrichtungen, welche Druck- bzw. Saugbewegungen des Spritzenkolbens (4) auslösen, eine Öffnung (9) für einen das Abzugelement (8) betätigenden Finger aufweist.

2. Dosiereinrichtung nach Anspruch 1, **dadurch gekennzeichnet, dass** das Abzugelement (8) ein federbelastetes Rastelement (13) aufweist, welches bei Betätigung des Abzugelementes (8) mit feststehenden Tastmarken oder Rastkerben (14) des Griffelementes (7) hörbar und fühlbar zusammenwirkt.

3. Dosiereinrichtung nach Anspruch 2, **dadurch gekennzeichnet, dass** die Tastmarken bzw. Rastkerben (14) auf der Innenseite eines im Griffelement (7) integrierten Bügels (15) angeordnet sind, welcher an die Bewegungsbahn des Abzugelementes (8) angepasst ist.

4. Dosiereinrichtung nach einem der Ansprüche 1 bis 3, **dadurch gekennzeichnet, dass** das Getriebe (10) aus einem am Abzugelement (8) fixierten Zahnrad (11) besteht, welches in eine am verschiebbaren Betätigungselement (5) für den Spritzenkolben (4) befestigte Zahnstange (12) eingreift.

5. Dosiereinrichtung nach einem der Ansprüche 1 bis 4, **dadurch gekennzeichnet, dass** das Griffelement (7) eine Führung (17) zur Aufnahme einer längs der Achse der Kolbenspritze (2) verschiebbaren Halterung (18) für den auf die Kolbenspritze (2) aufsteckbaren Embryotransfer-Katheter (3) aufweist.

6. Dosiereinrichtung nach Anspruch 5, **dadurch gekennzeichnet, dass** für die Festlegung der Halterung (18) des Embryotransfer-Katheters (3) im Griffelement (7) eine fingerbetätigbare Fixiereinrichtung (19) vorgesehen ist.

7. Dosiereinrichtung nach einem der Ansprüche 1 bis 6, **dadurch gekennzeichnet, dass** im Griffelement (7) eine in Richtung des Embryotransfer-Katheters (3) wirkende, batteriebetriebene Beleuchtungseinrichtung (20) integriert ist.

8. Dosiereinrichtung nach einem der Ansprüche 1 bis 7, **dadurch gekennzeichnet, dass** das Griffelement (7) aus zwei miteinander verschraubbaren Kunststoff-Spritzgussteilen (7a, 7b) besteht, an welchen die Halteeinrichtung (1) für die Kolbenspritze (2) mittels Verschraubung (21) oder Verklebung befestigt ist.

## Claims

1. Dispensing device for uterine embryo transfer with a holding means (1) for a plunger syringe (2), which is connectable to the proximal end of an embryo transfer catheter (3), the plunger (4) of the plunger syringe (2) being detachably attached to a shiftable actuating element (5), and the holding means (1) of the plunger syringe (2) being attached to a gripping element (7), which can be gripped with one hand and in which a finger-actuated trigger element (8) is rotatably borne, said trigger element (8) being connected via a gear (10) to the shiftable actuating element (5) of the plunger (4), **characterised in that** the trigger element (8) has an opening (9) for the finger actuating the trigger element (8), such that rotary motion is enabled in both directions of rotation, initiating a pressing or a sucking movement of the plunger (4).

2. Dispensing device according to claim 1, **characterised in that** the trigger element (8) is provided with a spring-loaded locking element (13), which interacts with fixed palpable marks or locking notches (14) of the gripping element (7) in a way that can be heard and felt when the trigger element (8) is actuated.

3. Dispensing device according to claim 2, **characterised in that** the palpable marks or locking notches (14) are placed on the inside of a bow (15) integrated in the gripping element (7), said bow (15) corresponding to the path of motion of the trigger element (8).

4. Dispensing device according to any of claims 1 to 3, **characterised in that** the gear (10) comprises a toothed wheel (11) fixed on the trigger element (8), which meshes with a toothed rack (12) attached to the shiftable actuating element (5) for the plunger (4).

5. Dispensing device according to any of claims 1 to 4, **characterised in that** the gripping element (7) is provided with a guide surface (17) for receiving a holding element (18) for the embryo transfer catheter (3) attached to the plunger syringe (2), which holding element (18) can be shifted along the axis of the plunger syringe (2).

6. Dispensing device according to claim 5, **characterised in that** for arresting the holding element (18) of the embryo transfer catheter (3) a finger-actuated arresting means (19) is provided on the gripping element (8).

7. Dispensing device according to any of claims 1 to 6, **characterised in that** a battery-powered lighting device (20) acting in the direction of the embryo transfer catheter (3) is integrated in the gripping element (7).

8. Dispensing device according to any of claims 1 to 7, **characterised in that** the gripping element (7) consists of two injection-moulded plastic parts (7a, 7b) which are screwed together, onto which the holding means (1) for the plunger syringe (2) is either screw-mounted (21) or bonded.

## Revendications

1. Installation de dosage pour le transfert d'embryons dans l'utérus comportant une installation de fixation (1) pour une seringue à piston (2) dont l'extrémité proximale peut être reliée à un cathéter (3) de transfert d'embryons,
- le piston (4) de la seringue (2) étant fixé de manière amovible à un élément d'actionnement (5), coulissant,
- l'installation de fixation (1) de la seringue à piston (2) étant fixée à un élément de poignée (7) qui se prend à la main, et dans lequel une gâchette (8) actionnée par les doigts est montée pivotante, et
- la gâchette (8) est reliée par une transmission (10) à l'élément d'actionnement (5), coulissant, du piston de seringue (4),
**caractérisée en ce que**
pour soutenir les mouvements de rotation dans les deux directions déclenchant des mouvements de poussée ou d'aspiration du piston de seringue (4), la gâchette (8) comporte une ouverture (9) pour un doigt actionnant la gâchette (8).

2. Installation de dosage selon la revendication 1,
**caractérisée en ce que**
la gâchette (8) comporte un élément d'encliquetage (13) chargé par un ressort qui, lors de l'actionnement de la gâchette (8), coopère de manière sonore et tactile avec des repères de palpage, fixes ou des encoches d'encliquetage (14) de l'élément de poignée (7).

3. Installation de dosage selon la revendication 2,
**caractérisée en ce que**
les repères de palpage ou les encoches d'encliquetage (14) sont prévus sur le côté intérieur de l'arceau (15) intégré à l'élément de poignée (7) et qui est adapté à la trajectoire de la gâchette (8).

4. Installation de dosage selon les revendications 1 à 3,
**caractérisée en ce que**
la transmission (10) se compose d'un pignon denté (11) fixé à la gâchette (8) et en prise dans un élément d'actionnement (5) coulissant d'une crémaillère (12) fixée au piston (4) de la seringue.

5. Installation de dosage selon les revendications 1 à 4,
**caractérisée en ce que**
l'élément de poignée (7) comporte un moyen de guidage (17) pour recevoir un moyen de fixation (18) coulissant le long de l'axe de la seringue à piston (2) pour le cathéter de transfert d'embryons (3) qui s'emmanche sur la seringue à piston (2).

6. Installation de dosage selon la revendication 5,
**caractérisée par**
une installation de fixation (19) actionnée par un doigt pour fixer le moyen de fixation (18) du cathéter de transfert d'embryons (3) dans l'élément de poignée (7).

7. Installation de dosage selon les revendications 1 à 6,
**caractérisée en ce qu'**
une installation d'éclairage (20) à batterie est intégrée dans l'élément de poignée (7), pour éclairer dans la direction du cathéter de transfert d'embryons (3).

8. Installation de dosage selon les revendications 1 à 7,
**caractérisée en ce que**
l'élément de poignée (7) se compose de deux pièces en matière plastique injectée (7a, 7b) qui se vissent l'une à l'autre et auxquelles est fixée une installation de fixation (1) de la seringue à piston (2) par des moyens de vissage (21) ou par collage.
